# EUROPEAN PATENT SPECIFICATION

(11) **EP 0 812 587 B1**
(45) Date of publication and mention of the grant of the patent: **11.09.2002**
(21) Application number: 96304460.7
(22) Date of filing: 14.06.1996
(51) Int. Cl.: A61K 9/20, A61K 9/06, A61K 9/08, A61K 9/10

(54) **Transdermal compositions containing Nimesulide**
Transdermale Zusammensetzungen enthaltend Nimesulide
Compositions transdermales contenant de la Nimesulide

(43) Date of publication of application: 17.12.1997
(73) Proprietor: Panacea Biotec Limited, New Dehli 110 001 (IN)
(72) Inventor: Jain, Rajesh, Mr., New Delhi 110 001 (IN); Singh, Amarjit Dr., New Delhi 110 001 (IN)
(74) Representative: Matthews, Derek Peter

(56) References cited:
- EP-A- 0 532 900
- WO-A-91/17774
- WO-A-96/11002
- JOURNAL OF CONTROLLED RELEASE, vol. 25, no. 1 / 02, 27 May 1993, pages 1-20, XP000361364 SANTUS G C ET AL: "TRANSDERMAL ENHANCER PATENT LITERATURE"

## Description

This invention relates to novel therapeutic anti-inflammatory and analgesic pharmaceutical compositions containing Nimesulide which is N-(4 nitro, 2 phenoxyphenyl)methane sulphonamide for use transdermally and a process for the manufacture thereof.

### BACKGROUND OF THE INVENTION

For a drug to be absorbed transdermally, it has to travel through various layers of the skin before reaching the site of action.

The layers of the skin are different in nature some are hydrophilic while some are lipophilic (Montagna W. Parrakhal PF: The structure and Function of the skin, 3rd ed. Academic press, New York, 1974). Accordingly, any drug which is used transdermally must possess both hydrophilic and lipophilic properties. Nimesulide which is N-(4 nitro, 2 phenoxyphenyl) methane sulphonamide, is a highly hydrophobic drug and consequently it is considered a poor candidate for transdermal absorption. When applied to the skin, it is absorbed in very minute quantities or not absorbed at all.

Transdermal routes for administration of anti-inflammatory agents offer various advantages over the oral route such as lower dosage, less toxicity/side effects, no G I irritation, no dose dumping in the body and it is more site specific (Chien YW: Novel Drug Delivery System, Marcel Dekker, New York, 1982).

The literature and market surveys show that at present, there exists no properly effective percutaneous formulations of Nimesulide.

In the patent for Nimesulide drug molecule (US Patent No. 3,840,597) the use of Nimesulide as an anti-inflammatory agent in the dose range of 1 mg to 500 mg per kg. body weight in the form of cream, gel, tapes and the like has been cited. According to our studies, it was observed that the drug either precipitated out in the conventional formulation or precipitated on application to human skin when applied as a conventional gel or cream in the above stated dosage and practically no percutaneous absorption occured. An over-riding difficulty is the inherent insolubility of the Nimesulide in aqueous media and hence the provision of a dosage form which can contain Nimesulide in sufficiently high concentration to permit convenient use and yet meet the required criteria in terms of bioavailability e.g. enabling effective absorption through the skin.
U.S. Patent 5,446,070 granted to Mantell et al. discloses a flexible, finite, bioadhesive composition. The present invention however, is not a finite composition or bioadhesive composition. The present invention comprises a composition which is non-finite in other words capable of being applied to large body areas for action at site of inflammation for instance the synovial fluids in joints.

Secondly, where the Mantell patent is restricted to a solvent range of 5 to 70 wt%, the solvent concentration of the present invention is upto 99.99% since the composition of the invention is a spreading non-finite composition.

Besides, unlike the Mantell et al. Patent which requires a plasticiser, a plasticiser is not required in the present invention because of its non-utility.

As regards the presence of polysaccharide, the present invention uses it along with a non-polysaccharide and the usage of a polysaccharide is at a much lower % by weight than 20% to 50% by weight as disclosed by Mantell.

Besides the polysaccharide used by Mantell in large quantities is necessary for bioadhesion which is not required in our case.

Also Mantell discloses water as an non-entity whereas in the present invention water is required in the composition.

The use of Nimesulide through intra-muscular administration as an analgesic agent has not been successful because Nimesulide is practically insoluble in water and its formulations in conventional oily bases or as suspensions result in depot formation in the muscular tissues which defies the main objective of quick relief.

The market and literature survey shows that no parenteral dosage form of Nimesulide is reported (Drugs 48(3) 431-454, 1994).

EP-782855 discloses an anti-inflammatory agent for external application containing Nimesulide as the active ingredient dispersed in a base component, but not in a soluble, penetrable form.

It is an object of the present invention to provide a therapeutic composition containing Nimesulide in combination with other compounds which alter the hydrophobic property of Nimesulide, and a process for the manufacture thereof thus making it possible for the said composition to be used for direct application on the skin for the treatment of inflammation through transdermal absorption.

It is a further object of the present invention to provide a novel therapeutic composition containing Nimesulide in combination with other compounds which alter the physico-chemical properties of Nimesulide, thus making it possible for the said composition to be used for direct application to the skin for the treatment of inflammation through transdermal absorption, at dose levels much lower than the dose levels according to the known art.

### SUMMARY OF THE INVENTION

The present invention provides a Novel Therapeutic Anti-inflammatory and Analgesic pharmaceutical composition for topical and/or
transdermal use which comprises:

| | |
|---|---|
| nimesulide (in solubilised form) | 0.1% to 10% w/w |
| percutaneous absorption enhancing vehicle base | 90% to 99.9% w/w |

wherein the said percutaneous enhancing vehicle base acts as a microcarrier preconcentrate or a microcarrier and comprises:

| | |
|---|---|
| percutaneous enhancer | 0.5% to 60% w/w |
| surfactant | 0.0% to 12% w/w |
| Gelling agent/ thickening agent | 0.2 to 19% w/w |
| one or more vehicles/ bases including water | 5% to 97% w/w |

and wherein said percutaneous enhancer is selected from the group consisting of vaccenic, cis-vaccenic, linoleic, linolenic, elaidic, oleic, pretroselinic, erucic or nervonic acid and/or any of their corresponding alcohols, or 1-dodecylazacycloheptane-2-one, or sulphoxides such as dimethylsulphoxide, n-decyl methylsulphoxide, or dimethylacetamide, dimethylformamide, N,N-diethyl-m-toluamide, or pyrrolidones such as 2-pyrrolidone and N-methyl-2-pyrrolidone.

Preferably the percutaneous enhancing base comprises :

| | |
|---|---|
| Percutaneous enhancer as herein described. | 6% to 15% w/w |
| Surfactant as herein described | 0.5% to 12% w/w |
| Gelling agent/Thickening agent as herein described | 0.5% to 19% w/w |
| One or more vehicle/base including water as herein described | 5% to 60% w/w |

Water is required for the composition in the range of 1% to 15% w/w, preferably 9% to 11% w/w and more preferably in the range of 9.5% to 10.5% w/w.

Besides the above disclosed ingredients the composition for topical use may also comprise a neutralising agent/pH adjusting agent such as herein described in the range of 0.0% to 2.0%.

### DETAILED DESCRIPTION OF THE INVENTION

According to the present invention, it has been found that it is possible to solubilise and deliver a highly hydrophobic drug like Nimesulide to the site of action through transdermal route. The present invention involves the process of incorporation of Nimesulide in a formulation which can solubilize the drug and transport it through the skin barriers, in intact condition to the site of action.

The present invention concerns a therapeutic anti-inflammatory and analgesic pharmaceutical composition for topical and/or transdermal use which comprises:

| | |
|---|---|
| nimesulide (in solubilised form) | 0.1% to 10% w/w |
| percutaneous absorption enhancing vehicle base | 90% to 99.9% w/w |

wherein the said percutaneous enhancing vehicle base acts as a microcarrier preconcentrate or a microcarrier and comprises:

| | |
|---|---|
| percutaneous enhancer | 0.5% to 60% w/w |
| surfactant | 0.0% to 12% w/w |
| Gelling agent/ thickening agent | 0.2 to 19% w/w |
| one or more vehicles/ bases including water | 5% to 97% w/w |

and wherein said percutaneous enhancer is selected from the group consisting of vaccenic, cis-vaccenic, linoleic, linolenic, elaidic, oleic, pretroselinic, erucic or nervonic acid and/or any of their corresponding alcohols, or 1-dodecylazacycloheptane-2-one, or sulphoxides such as dimethylsulphoxide, n-decyl methylsulphoxide, or dimethylacetamide, dimethylformamide, N,N-diethyl-m-toluamide, or pyrrolidones such as 2-pyrrolidone and N-methyl-2-pyrrolidone.

Preferably the percutaneous enhancing base comprises :

| | |
|---|---|
| Percutaneous enhancer as herein described | 6% to 15% w/w |
| Surfactant as herein described | 0.5% to 12% w/w |
| Gelling agent/Thickening agent as herein described | 0.5% to 19% w/w |
| One or more vehicles bases including water as herein described | 5% to 60% w/w |

Preferably nimesulide is in the range of 1% to 5% w/w.

More preferably the composition for topical use also comprises a Neutralising agent/pH adjusting agent as herein described in the range of 0.0% to 2.0%.

The novel Therapeutic Anti-Inflammatory and Analgesic Composition for topical use according to the present invention, is prepared by a process which comprises the following steps:
(a) 0.5% to 30% w/w of a Percutaneous enhancer, as herein described, is mixed with 2.5% to 30% w/w of one or more Vehicles or bases, as herein described, in a container by stirring and to the mixture obtained 0.1% to 10% w/w of Nimesulide is added and stirred till completely dissolved.
(b) 0.5% to 12% w/w of a Surfactant, as herein described, 0.2% to 50% w/w of a Gelling agent/thickening agent, as herein described, and 2.5% to 30% w/w of one or more Vehicles/Bases as herein described, are mixed in a homogeniser to obtain a homogenised mixture.
(c) The mixture obtained in step (a) is added to the homogenised mixture obtained in step (b) under stirring without vortex formation to avoid aeration. The mixture is neutralised or its pH adjusted by addition of 0.0% to 2.0% of a neutralising agent or a pH adjusting agent, as herein described, with slow stirring resulting in the preparation of the desired Anti-inflammatory and Analgesic Composition.

The Percutaneous enhancer is selected from the group consisting of vaccenic, cis-vaccenic, Linoleic, Linolenic, elaidic, oleic, petroselinic, erucic or nervonic acid and/or any of their corresponding alcohols, or 1-dodecylazacycloheptane-2-one also known as azone; sulphoxides like dimethylsuphoxide, n-decyl methylsulphoxide; dimethylacetamide, dimethylformamide N, N-diethyl-m-toluamide or Pyrrolidones like 2-pyrrolidone and N-methyl-2 Pyrrolidone.

As a surfactant, any pharmaceutically acceptable hydrophilic or lipophilic surfactant or mixture thereof may be used, especially suitable for this purpose are the reaction products of natural or hydrogenated vegetable oils and ethylene glycol i.e. polyoxyethylene glycolated natural or hydrogenated vegetable oils, e.g. polyoxyethylene glycolated natural or hydrogenated castor oils; especially various tensides available under the trade name CREMOPHOR particularly CREMOPHOR RH 40 and CREMOPHOREL. Also suitable for use are the various surfactants available under the trade name NIKKOL e.g. NIKKOL HCO-60.

Polyoxyethylene Sorbitan fatty acid esters e.g. mono and trilauryl, palmityl, stearyl and oleyl esters e.g. those available under the trade name TWEEN preferably TWEEN 40 and TWEEN 80.

Polyoxyethylene-polyoxypropylene block copolymers e.g. especially those available under the trade name POLOXAMER preferably POLOXAMER 188.

Polyoxethylene fatty acid esters, for example polyoxyethylene stearic acid esters, commercially available under the trade name MYRJ as well as polyoxyethylene fatty acid esters commercially available under the trade name CEIIOL HE;
Propylene glycol mono-and di-fatty acid esters such as propylene glycol dicaprylate, propylene glycol dilaurate, propylene glycol hydroxystearate, propylene glycol isostearate, propylene glycol laurate, propylene glycol ricinoleate, propylene glycol stearate; Examples of suitable lipophilic surfactants include Transesterification products of natural Vegetable oil triglycerides and polyalkylene polyols. Preferred are products obtained by trans-estrification of 2 molar parts of natural vegetable oil triglycerides with one molar parts of polyethylene glycol (e.g. having an average molecular weight of from 200 to 800). Various forms of such trans-esterification product are commercially available under the trade name LABRAFIL, preferably LABRAFIL M 1944 CS;

Sorbitan fatty acid esters commercially available under the trade name SPAN including Sorbitan monolauryl, monopalmityl, monostearyl, tristearyl, monooleyl and trioleyl esters; Monoglycerides e.g. Glycerol monooleate, glycerol monopalmitate and glyceryl 1 monostearate commercially available under the trade names MYVATEX, MYVAPIEX and MYVEROL.

As Gelling Agent/Thickening agent, any known such pharmaceutically acceptable agent may be used including synthetic or semi-synthetic polymeric materials, polyacrylate and polyacrylate co-polymeric resins e.g. polyacrylic acid and polyacrylic acid/methacrylic acid resins, commercially available under the trade name CARBOPOL, particularly CARBOPOL 934, 940 and 941 and EUDRAGIT, particularly EUDRAGIT E, L, S, SL and RS; Cellulose and cellulose derivatives including alkyl celluloses e.g. methyl-, ethyl- and propyl-celluloses; hydroxyalkyl-celluloses e.g. hydroxypropyl cellulose, hydroxypropyl alkylcellulose such as hydroxypropyl-methyl- cellulose, acylated celluloses e.g. cellulose-acetates, cellulose acetate phthalates and salts thereof such as sodium carboxymethyl cellulose; Polyvinyl resins including polyvinylacetates and alcohols as well as other polymeric materials including alginates e.g. alginic acid and salts thereof e.g. sodium alginate and propylene glycol alginate.

As a Neutralising/pH adjusting agent any such conventional agent may be used including sodium bicarbonate, sodium hydroxide, potassium hydroxide, borax, disodium hydrogen phosphate and sodium dihydrogen phosphate. Preferably polar organic amines like diethylamine, diisopropanolamine, triethylamine and triethanolamine may be used.

As vehicles/base, the following may be used :

Pharmaceutically acceptably lower (having C₁₋₅) alkanols, particularly ethanol; water soluble macrogols like polyethylene glycol having an average molecular weight from 200 to 600 : 1,2-propylene carbonate, propane-1,2-diol and 1,2 -propylene glycol; glycerol triacetate or (1,2,3) -triacetin; lower ketones, particularly acetone and 1,2,3 -propanetriol may be incorporated.

Water in varying concentration may be added to provide requisite hydrophilic nature to the composition.

Pharmaceutically acceptable C₁₋₅ alkyl or tetra hydrofurfuryl; di or partial ether of a low molecular weight mono or polyoxy-alkanediol particularly those available under the trade names

### TRANSCUTOL and COLYCOFUROL.

As base having lipophilic phase for the preparation of emulsions, may be used fatty acid triglycerides, preferably medium chain fatty acid triglycerides; vegetable oils like coconut oils, olive oil, castor oil and their derivatives; ethyl oleate.

As base, for the preparation of the said therapeutic composition in the form of an ointment, may be used fatty acids, fats, oils and waxes of animal origin like bees wax, spermacetii, wool fat, waxes of vegetable origin or mineral origin like hard, soft and liquid paraffin.

The topical dosage forms are formulated suitably such that the resultant product is easy to apply and is non-staining.

For the said therapeutic composition in the form of aerosol formulation for topical applications, as pharmaceutically acceptable propellants may be used chlorofluoro-carbons e.g. the Propellant 11, Propellant 12, Propellant 114; Hydrocarbon propellants like n-butane, isobutane and propane; compressed gas propellants e.g. Nitrous oxide, carbon dioxide, and nitrogen.

The novel therapeutic composition according to the present invention may be used in the following forms:
1. Topical aqueous gel.
2. Oil-in-water or water-in-oil emulsion or micro-emulsion or cream.
3. Solution for topical applications.
4. Ointment.
5. Aerosol formulation for topical applications.

The therapeutic composition according to the present invention may be applied on the skin by utilising a physical form of energy like electrical energy or ultrasonic energy to effect better percutaneous absorption of the drug.

The invention will now be described with reference to the foregoing examples :

### Example 1

| Preparation of topical gel dosage form | | |
|---|---|---|
| Sl. No. | Component | Quantity |
| 1, | Nimesulide | 2.0 g |
| 2. | Dimethyl acetamide | 22.0 g |
| 3. | Ethyl Alcohol | 40.0 g |
| 4. | Acetone | 10.0 g |
| 5. | Cremophor® RH 40 | 4.0 g |
| 6. | Propylene glycol | 38.0 g |
| 7. | Polyethylene glycol 400 | 48.8 g |
| 8. | Carbopol® 934 | 4.0 g |
| 9. | Water | 30.0 g |
| 10. | Diethylamine | 1.2 g |
| | Total | 200.0 g |

- Step (a): Dimethylacetamide is mixed with ethyl alcohol and acetone at 30°C in a container with stirring. To the mixture obtained Nimesulide is added and stirred till completely dissolved.
- Step (b): Propylene glycol, polyethylene glycol 400 and water are mixed in homogenizer. To the homogenised mixture obtained, 1.5% w/w of carbopol 934 is added in small amounts at a time at room temperature and the speed of the homogenizer is kept at approximately 1500-2000 rpm.
- Step (c): The mixture obtained in step (a) is added to the mixture obtained in step (b) under stirring without vortex formation to avoid aeration preferably under vacuum (25 mm of Hg). The mixture obtained is neutralised by slow addition of Diethylamine with slow stirring at a temperature of 25°-30°C and under vacuum (25 mm of Hg) to affect gel formation.

### Example 2

| Preparation of emulsion type topical dosage form. | | |
|---|---|---|
| Sl. No. | Component | Quantity |
| 1. | Nimesulide | 1.0 g |
| 2. | Transcutol® | 35.0 g |
| 3. | Water | 10.0 g |
| 4. | Disodium hydrogen phosphate | 0.1 g |
| 5. | Cremophor® RH 40 | 5.0 g |
| 6. | Labrafil® M 1944 CS | 10.0 g |
| 7. | Glyceryl monostearate | 8.0 g |
| 8. | Stearic acid | 13.0 g |
| 9. | Ethyl oleate | 2.9 g |
| 10. | Diethyl sulphoxide | 15.0 g |
| | Total | 100.0 g |

Dissolve Nimesulide in a mixture of (6), (7), (8), (9) and (10) with warming. Separately mix (2), (3), (4) and (5) and slowly add the Nimesulide mixture to it with stirring.

### Example 3

| Preparation of a solution type dosage form for topical application | | |
|---|---|---|
| Sl. No. | Component | Quantity |
| 1. | Nimesulide | 1.0 g |
| 2. | Dimethyl formamide | 10.0 g |
| 3. | Poloxamer® 188 | 2.0 g |
| 4. | Ethyl alcohol | 20.0 g |
| 5. | Propylene glycol | 25.0 g |
| 6. | Polyethylene glycol 400 | 42.0 g |
| 7. | Hydroxypropylmethylcellulose | 1.0 g |
| 8. | Triethanolamine | 0.2 g |
| 9. | Water | 1.0 g |
| | Total | 100.0 g |

Nimesulide is dissolved in (2) with stirring and (3), (4), (5), (6), (7) and (8) are added to obtain a clear solution with stirring.

### Example 4

| Preparation of ointment type dosage form topical application. | | |
|---|---|---|
| Sl. No. | Component | Quantity |
| 1. | Nimesulide | 2.0 g |
| 2. | Dimethylsulphoxide | 21.0 g |
| 3. | Glycerylmonostearate | 16.0 g |
| 4. | Mineral Oil | 62.0 g |
| 5. | White petrolatum | 97.0 g |
| 6. | Water | 2.0 g |
| | Total | 200.0 g |

Warm (3), (4) and (5) and add with stirring a solution of Nimesulide in dimethyl sulphoxide.

### Example 5

| Preparation of an aerosol dosage form for topical use. | | |
|---|---|---|
| Sl. No. | Component | Quantity |
| 1. | Nimesulide | 1.0 g |
| 2. | Dimethylacetamide | 10.0 g |
| 3. | Ethyl Alcohol | 10.0 g |
| 4. | Cremophor® RH 40 | 10.0 g |
| 5. | Propellant 114 | 29.0 g |
| 6. | Propellant 12 | 39.0 g |
| 7. | Water | 1.0 g |
| | Total | 100.0 g |

The analgesic activity of the therapeutic composition, prepared according to the present invention, was found to be dose dependent and passed the tests of subacute toxicity and undue toxicity.

The dose levels of the novel Anti-inflammatory and Analgesic composition, according to the present invention, are comparatively much lower than the dose levels of the Conventional Nimesulide formulations for equally effective results.

The various forms of the therapeutic composition prepared according to the present invention were subjected to in-vitro drug release studies using modified USP dissolution apparatus attached with enhancer cell (Pharm Tech. Jan. 1995, 52-58). The dissolution media used was phosphate buffer pH 7.4. The results indicated that the cumulative drug release and permeation flux were proportional to the drug load.

The said compositions were also subjected to standard pharmacological test methods to measure anti-inflammatory activity such as rat paw oedema and guinea pig erythema. These tests showed significant activity when compared to placebo.

The said Therapeutic compositions were also tested on sixty healthy human volunteers for irritation or other side effects. No incidence of irritation/side effects was reported.

## Claims

1. A therapeutic anti-inflammatory and analgesic pharmaceutical composition for topical and/or transdermal use which comprises:
| | |
|---|---|
| nimesulide (in solubilised form) | 0.1% to 10% w/w |
| percutaneous absorption enhancing vehicle base | 90% to 99.9% w/w |
wherein the said percutaneous enhancing vehicle base acts as a microcarrier preconcentrate or a microcarrier and comprises:
| | |
|---|---|
| percutaneous enhancer | 0.5% to 60% w/w |
| surfactant | 0.0% to 12% w/w |
| Gelling agent/ thickening agent | 0.2 to 19% w/w |
| one or more vehicles/ bases including water | 5% to 97% w/w |
and wherein said percutaneous enhancer is selected from the group consisting of vaccenic, cis-vaccenic, linoleic, linolenic, elaidic, oleic, pretroselinic, erucic or nervonic acid and/or any of their corresponding alcohols, or l-dodecylazacycloheptane-2-one, or sulphoxides such as dimethylsulphoxide, n-decyl methylsulphoxide, or dimethylacetamide, dimethylformamide, N,N-diethyl-m-toluamide, or pyrrolidones such as 2-pyrrolidone and N-methyl-2-pyrrolidone.

2. A pharmaceutical composition as claimed in claim 1 wherein said percutaneous enhancing vehicle base comprises:
| | |
|---|---|
| percutaneous enhancer : | 6% to 15% w/w |
| surfactant | 0.5% to 12% w/w |
| gelling agent/ thickening agent | 0.5% to 19% w/w |
| one or more vehicles/ bases including water | 5% to 60% w/w |
wherein said percutaneous enhancer is selected from the group consisting of vaccenic, cis-vaccenic, linoleic, linolenic, elaidic, oleic, pretroselinic, erucic or nervonic acid and/or any of their corresponding alcohols, or 1-dodecylazacycloheptane-2-one, or sulphoxides such as dimethylsulphoxide, n-decyl methylsulphoxide, or dimethylacetamide, dimethylformamide, N,N-diethyl-m-toluamide, or pyrrolidones such as 2-pyrrolidone and N-methyl-2-pyrrolidone.

3. A pharmaceutical composition as claimed in either claim 1 or claim 2 which further comprises a neutralising agent/pH adjusting agent in the range of 0.0% w/w to 2.0% w/w.

4. A pharmaceutical composition as claimed in any one of claims 1-3 wherein the percutaneous enhancer is dimethylacetamide.

5. A pharmaceutical composition as claimed in any one of claims 1 to 4 wherein the said surfactant is selected from the group consisting of:
any pharmaceutically acceptable hydrophilic or lipophilic surfactant or mixture thereof, the reaction products of natural or hydrogenated vegetable oils and ethylene glycol such as polyoxyethylene glycolated natural or hydrogenated vegetable oils such as polyoxyethylene glycolated natural or hydrogenated castor oils; polyoxyethylene-sorbitan fatty acid esters e.g. mono and trilauryl, pamityl, stearyl and oleyl esters, polyoxyethylene-polyoxypropylene block copolymers, polyoxyethylene fatty acid esters, propylene glycol mono- and di-fatty acid esters such as propylene glycol dicaprylate, propylene glycol dilaurate, propylene glycol hydroxystearate, propylene glycol isostearate, propylene glycol laurate, propylene glycol ricinoleate, propylene glycol stearate; lipophilic surfactants such as transesterification products of natural vegetable oil triglycerides and polyalkylene polyols, sorbitan fatty acid esters, monoglycerides such as glycerol monooleate, glycerol monopalmitate and glyceryl monostearate.

6. A pharmaceutical composition as claimed in any one of claims 1 to 5 wherein said gelling/thickening agent is selected from the group consisting of:
synthetic or semi-synthetic polymeric materials, polyacrylate and polyacrylate co-polymeric resins such as polyacrylic acid and polyacrylic acid/methacrylic acid resins, cellulose and cellulose derivatives including alkyl celluloses such as methyl-, ethyl- and propyl-celluloses; hydroxyalkyl-celluloses such as hydroxypropyl cellulose, hydroxypropyl alkylcellulose such as hydroxypropyl-methyl-cellulose, acylated celluloses such as cellulose-acetates, cellulose acetate phthalates and salts thereof such as sodium carboxymethyl cellulose; polyvinyl resins including polyvinylacetates and alcohols as well as other polymeric materials including alginates such as alginic acid and salts thereof such as sodium alginate and propylene glycol alginate.

7. A pharmaceutical composition as claimed in claim 2 wherein water is present in the range 1% to 15% w/w.

8. A pharmaceutical composition as claimed in any one of claims 1 to 7 wherein water is present in the range of 9% to 11%.

9. A pharmaceutical composition as claimed in claim 8 wherein water is present in the range of 9.5% to 10.5% w/w.

10. A pharmaceutical composition as claimed in any one of claims 3 to 9 wherein the said neutralising agent is selected from the group consisting of:
sodium bicarbonate, sodium hydroxide, potassium hydroxide, borax, disodium hydrogen phosphate and sodium dihydrogen phosphate, polar organic amines such as diethylamine, diisopropanolamine, triethylamine and triethanolamine.

11. A pharmaceutical composition as claimed in any one of claims 1 to 10 wherein the said vehicle/base is selected from the group consisting of:
pharmaceutically acceptable lower (having C₁₋₅) alkanols, particularly ethanol; water soluble macrogols like polyethylene glycol having an average molecular weight from 200 to 600 : 1,2-propylene carbonate, propane-1,2-diol and 1,2-propylene glycol; glycerol triacetate or (1,2,3)-triacetin; lower ketones, particularly acetone and 1,2,3-propanetriol, pharmaceutically acceptable C₁₋₅ alkyl or tetra hydrofurfuryl; di or partial ether of a low molecular weight mono or polyoxy-alkanediol; fatty acid triglycerides, preferably medium chain fatty acid triglycerides; vegetable oils like coconut oils, olive oil, castor oil and their derivatives; ethyl oleate, fats, oils and waxes of animal origin like bees wax, spermacetii, wool fat, waxes of vegetable origin or mineral origin like hard, soft and liquid paraffin.

12. A pharmaceutical composition as claimed in any one of claims 1 to 11 which is in the form of a topical aqueous gel.

13. A pharmaceutical composition as claimed in any one of claims 1 to 11 which is in the form of an oil-in-water or water-in-oil emulsion or micro-emulsion or cream.

14. A pharmaceutical composition as claimed in any one of claims 1 to 11 which is in the form of a solution for topical application.

15. A pharmaceutical composition as claimed in any one of claims 1 to 11 which is in the form of an ointment.

16. A pharmaceutical composition as claimed in any one of claims 1 to 11 which is in the form of an aerosol formulation.

17. A process for the production of a therapeutic anti-inflammatory and analgesic pharmaceutical composition for topical use which comprises the following steps:
a) mixing 0.5 w/w to 30% of a percutaneous enhancer with 2.5% to 30% w/w of one or more vehicles or bases;
wherein said percutaneous enhancer is selected from the group consisting of vaccenic, cis-vaccenic, linoleic, linolenic, elaidic, oleic, pretroselinic, erucic or nervonic acid and/or any of their corresponding alcohols, or 1-dodecylazacycloheptane-2-one, or sulphoxides such as dimethylsulphoxide, n-decyl methylsulphoxide, or dimethylacetamide, dimethylformamide, N,N-diethyl-m-toluamide, or pyrrolidones such as 2-pyrrolidone and N-methyl-2-pyrrolidone.
b) adding to the mixture of step a), 0.1% w/w to 10% w/w of nimesulide followed by stirring the mixture until completely dissolved;
c) mixing separately 0.5% w/w to 12% w/w of a surfactant, 0.2% w/w to 50% w/w of a gelling agent/thickening agent and 2.5% w/w to 30% w/w of one or more vehicles or bases and mixing the entire mixture in a homogeniser to obtain a homogenised mixture;
d) adding the mixture obtained in step b) to the homogenised mixture obtained in step c) under stirring to obtain the desired composition for analgesic use.

18. A process as claimed in claim 17 wherein a neutralising agent or a pH adjusting agent is added to the composition in step d) to neutralise or adjust the pH of the mixture.

19. A process as claimed in claim 18 wherein the said neutralising agent/pH adjusting agent is added in the range of 0.0% w/w to 2.0% w/w.

## Patentansprüche

1. Therapeutische antientzündliche und analgetische pharmazeutische Zusammensetzung zur lokalen und/oder transdermalen Verwendung mit:
| | |
|---|---|
| Nimesulid (in solubilisierter Form) | 0,1 % bis 10 % w/w |
| eine die perkutane Aufnahme fördernde Trägerstoffbasis | 90 % bis 99,9 % w/w |
wobei die die perkutane Aufnahme fördernde Trägerstoffbasis als ein Mikroträger-Vorkonzentrat oder ein Miktroträger wirkt und folgendes aufweist:
| | |
|---|---|
| perkutaner Verstärker | 0,5 % bis 60 % w/w |
| oberflächenaktive Substanz | 0,0 % bis 12 % w/w |
| Geliermittel/Dickungsmittel | 0,2 bis 19 % w/w |
| einen oder mehrere Trägerstoffe/ Grundstoffe einschließlich Wasser | 5 % bis 97 % w/w |
und wobei der perkutane Verstärker aus der aus Vaccensäure, cis-Vaccensäure, Linolsäure, Linolensäure, Elaidinsäure, Ölsäure, Petroselinsäure, Erucasäure oder Nervonsäure und/oder einem ihrer korrespondierenden Alkohole, oder 1-Dodecylazacycloheptan-2-on, oder Sulfoxiden, wie Dimethylsulfoxid, n-Decylmethylsulfoxid, oder Dimethylacetamid, Dimethylformamid, N,N-Diethyl-m-toluamid, oder Pyrrolidone, wie 2-Pyrrolidon und N-Methyl-2-pyrrolido,n bestehenden Gruppe ausgewählt ist.

2. Pharmazeutische Zusammensetzung nach Anspruch 1, wobei die die perkutane Aufnahme fördernde Trägerstoffbasis folgendes aufweist:
| | |
|---|---|
| perkutaner Verstärker | 6 % bis 15 % w/w |
| oberflächenaktive Substanz | 0,5 % bis 12 % w/w |
| Geliermittel/Dickungsmittel | 0,5 % bis 19 % w/w |
| einen oder mehrere Trägerstoffe/ Grundstoffe einschließlich Wasser | 5 % bis 60 % w/w |
wobei der perkutane Verstärker aus der aus Vaccensäure, cis-Vaccensäure, Linolsäure, Linolensäure, Elaidinsäure, Ölsäure, Petroselinsäure, Erucasäure oder Nervonsäure und/oder einem ihrer korrespondierenden Alkohole, oder 1-Dodecylazacloheptan-2-on, oder Sulfoxiden, wie Dimethylsulfoxid, n-Decylmethylsulfoxid, oder Diemthylacetamid, Dimethylformamid, N,N-Diethyl-m-toluamid, oder Pyrrolidonen, wie 2-Pyrrolidon und N-Methyl-2-pyrrolidon, bestehenden Gruppe ausgewählt ist.

3. Pharmazeutische Zusammensetzung nach Anspruch 1 oder Anspruch 2, die weiterhin ein Neutralisierungsmittel/pH-Einstellungsmittel in dem Bereich von 0,0 % w/w bis 2,0 % w/w aufweist.

4. Pharmazeutische Zusammensetzung nach einem der Ansprüche 1 bis 3, wobei der perkutane Verstärker Dimethylacetamid ist.

5. Pharmazeutische Zusammensetzung nach einem der Ansprüche 1 bis 4, wobei die oberflächenaktive Substanz aus der folgenden Gruppe ausgewählt ist:
jede beliebige pharmazeutisch zulässige bzw. brauchbare hydrophile oder lipophile oberflächenaktive Substanz oder Mischungen davon, die Reaktionsprodukte von natürlichen oder hydrierten Pflanzenölen und Ethylenglycol, wie mit Polyoxyethylen glycolierte natürliche und hydrierte Pflanzenöle, wie mit Polyoxyethylen glycolierte natürliche oder hydrierte Rizinusöle, Polyoxyethylen-Sorbitanfettsäureester z.B. Mono- und Trilaurylester, Palmitylester, Stearylester und Oleylester, Polyoxyethylen-Polyoxypropylen-Blockcopolymere, Polyoxyethylenfettsäureester, Propylenglycolmono- und -difettsäureester, wie Propylenglycoldicaprylat, Propylenglycoldilaurat, Propylenglycolhydroxystearat, Propylenglycolisostearat, Propylenglycollaurat, Propylenglycolrizinoleat, Propylenglycolstearat, lipophile oberflächenaktive Substanzen, wie Umesterungsprodukte von natürlichen Pflanzenöltriglyceriden und Polyalkylenpolyole, Sorbitanfettsäureester, Monoglyceride, wie Glycerolmonooleat, Glycerolmonopalmitat und Glycerylmonostearat.

6. Pharmazeutische Zusammensetzung nach einem der Ansprüche 1 bis 5, wobei das Gelierungs-/Dickungsmittel aus der folgenden Gruppe ausgewählt ist:
synthetische oder halbsynthetische Polymermaterialien, Polyacrylat und Polyacrylat-Copolymerharze, wie Polyacrylsäure und Polyacrylsäure/Methacrylsäureharze, Cellulose und Cellulosederivate, einschließlich Alkylcellulosen, wie Methyl-, Ethyl- und Propylcellulosen, Hydroxyalkylcellulosen wie Hydroxypropylcellulose, Hydroxypropylalkylcellulose, wie Hydroxypropylmethylcellulose, acylierte Cellulosen, wie Celluloseacetate, Celluloseacetatphthalate und Salze davon wie Natriumcarboxymethylcellulose; Polyvinylharze einschließlich Polyvinylacetate und -alkohole sowie andere Polymermaterialien, einschließlich Alginate, wie Alginsäure und Salze davon, wie Natriumalginat und Propylenglycolalginat.

7. Pharmazeutische Zusammensetzung nach Anspruch 2, wobei Wasser in dem Bereich von 1 % bis 15 % w/w vorhanden ist.

8. Pharmazeutische Zusammensetzung nach einem der Ansprüche 1 bis 7, wobei Wasser in dem Bereich von 9 % bis 11 % vorhanden ist.

9. Pharmazeutische Zusammensetzung nach Anspruch 8, wobei Wasser in dem Bereich von 9,5 % bis 10,5 % w/w vorhanden ist.

10. Pharmazeutischen Zusammensetzung nach einem der Ansprüche 3 bis 9, wobei das Neutralisierungsmittel aus der folgenden Gruppe ausgewählt ist:
Natriumbicarbonat, Natriumhydroxid, Kaliumhydroxid, Borax, Dinatriumhydrogenphosphat und Natriumdihydrogenphosphat, polare organische Amine, wie Diethylamin, Diisopropanolamin, Triethylamin, Triethanolamin.

11. Pharmazeutische Zusammensetzung nach einem der Ansprüche 1 bis 10, wobei der Trägerstoff/Grundstoff aus der folgenden Gruppe ausgewählt ist:
pharmazeutisch zulässige bzw. brauchbare niedrige (mit C₁₋₅) Alkanole, insbesondere Ethanol; wasserlösliche Macrogole, wie Polyethylenglycol, mit einem mittleren Molekulargewicht von 200 bis 600: 1,2-Propylencarbonat, Propan-1,2-diol und 1,2-Propylenglycol; Glyceroltriacetat oder (1,2,3)-Triacetin; niedrige Ketone, insbesondere Aceton und 1,2,3-Propantriol, pharmazeutisch zulässige bzw. brauchbare C₁₋₅-Alkyl- oder Tetrahydrofurfuryl; Diether oder Teilether eines Mono- oder Polyoxyalkandiols mit niedrigem Molekulargewicht; Fettsäuretriglyceride, vorzugsweise mittelkettige Fettsäuretriglyceride; pflanzliche Öle, wie Kokosöle, Olivenöl, Rizinusöl und ihre Derivate; Ethyloleat, Fette, Öle und Wachse aus tierischem Ursprung, wie Bienenwachs, Spermacetii, Wollfett, Wachse aus pflanzlichem Ursprung oder mineralischem Ursprung, wie Hart-, Weich- und Flüssigparaffin.

12. Pharmazeutische Zusammensetzung nach einem der Ansprüche 1 bis 11, die in Form eines lokal anwendbaren wäßrigen Gels vorliegt.

13. Pharmazeutische Zusammensetzung nach einem der Ansprüche 1 bis 11, die in der Form einer Öl-in-Wasser- oder Wasser-in-Öl-Emulsion oder Mikroemulsion oder Creme vorliegt.

14. Pharmazeutische Zusammensetzung nach einem der Ansprüche 1 bis 11, die in der Form einer Lösung für eine örtliche Anwendung vorliegt.

15. Pharmazeutische Zusammensetzung nach einem der Ansprüche 1 bis 11, die in der Form einer Salbe vorliegt.

16. Pharmazeutische Zusammensetzung nach einem der Ansprüche 1 bis 11, die in der Form einer Aerosolformulierung vorliegt.

17. Verfahren zur Herstellung einer therapeutischen antientündlichen und analgetischen pharmazeutischen Zusammensetzung zur lokalen Verwendung, das die folgenden Schritte umfaßt:
a) Mischen von 0,5 w/w bis 30 % eines perkutanen Verstärkers mit 2,5 % bis 30 % w/w von einem oder mehreren Trägerstoffen oder Grundstoffen,
wobei der perkutane Verstärker aus der aus Vaccensäure, cis-Vaccensäure, Linolsäure, Linolensäure, Elaidinsäure, Ölsäure, Petroselinsäure, Erucasäure oder Nervonsäure und/oder einem ihrer korrespondierenden Alkohole, oder 1-Dodecylazacycloheptan-2-on, oder Sulfoxiden, wie Dimethylsulfoxid, n-Decylmethylsulfoxid, oder Diemthylacetamid, Diemthylformamid, N,N-Diethyl-m-toluamid, oder Pyrrolidonen, wie 2-Pyrrolidon und N-Methyl-2-pyrrolidon, bestehenden Gruppe ausgewählt ist.
b) Zugeben von 0,1 % w/w bis 10 % w/w Nimesulid zu dem Gemisch aus Schritt a) und anschließendes Rühren des Gemisches bis zur vollständigen Lösung;
c) getrenntes Mischen von 0,5 % w/w bis 12 % w/w einer oberflächenaktiven Substanz, 0,2 % w/w bis 50 % w/w eines Geliermittels/Dickungsmittels und 2,5 % w/w bis 30 % w/w eines oder mehrerer Trägerstoffe oder Grundstoffe und Mischen des gesamten Gemisches in einem Homogenisator, um ein homogenisiertes Gemisch zu erhalten,
d) Zugeben des unter Schritt b) erhaltenen Gemisches zu dem in Schritt c) erhaltenen homogenisierten Gemisch unter Rühren, um die gewünschte Zusammensetzung für eine analgetische Verwendung zu erhalten.

18. Verfahren nach Anspruch 17, wobei der Zusammensetzung in Schritt d) ein Neutralisierungsmittel oder ein pH-Einstellungsmittel zugegeben wird, um den pH-Wert des Gemisches zu neutralisieren oder einzustellen.

19. Verfahren nach Anspruch 18, wobei das Neutralisierungsmittel/pH-Einstellungsmittel in dem Bereich von 0,0 % w/w bis 2,0 % w/w zugegeben wird.

## Revendications

1. Composition pharmaceutique thérapeutique anti-inflammatoire et analgésique pour une utilisation topique et/ou transdermale qui comprend :
| | |
|---|---|
| de la nimésulide (sous forme soluble) | de 0,1 % à 10 % m/m |
| une base de véhicule amplificatrice d'absorption percutanée | de 90 % à 99,9 % m/m |
dans laquelle ladite base de véhicule amplificatrice percutanée agit comme un préconcentré de microporteur ou comme un microporteur et comprend :
| | |
|---|---|
| un amplificateur percutané | de 0,5 % à 60 % m/m |
| un tensio-actif | de 0,0 % à 12 % m/m |
| un gélifiant / un agent épaississant | de 0,2 % à 19 % m/m |
| un ou plusieurs véhicules / bases comprenant de l'eau | de 5 % à 97 % m/m |
et dans laquelle ledit amplificateur percutané est choisi dans le groupe constitué d'acide vaccénique, cis-vaccénique, linoléique, linolénique, élaïdique, oléique, pétrosélinique, érurique ou nervonique et/ou de n'importe lequel de leurs alcools correspondants, ou de dodécylazacyclo - 1 heptane-2-one ou de sulphoxydes tels que le diméthylsulfoxyde, le n-décylméthylsulphoxyde ou de diméthylacétamide, de diméthylformamide, de N,N-diéthyl-m-toluamide, ou de pyrrolidones telles que la 2-pyrrolidone et la N-méthyl-2-pyrrolidone.

2. Composition pharmaceutique selon la revendication 1 dans laquelle ladite base de véhicule amplificatrice percutanée comprend :
| | |
|---|---|
| un amplificateur percutané | de 6 % à 15 % m/m |
| un tensio-actif | de 0,5 % à 12 % m/m |
| un gélifiant / un agent épaississant | de 0,5 % à 19 % m/m |
| un ou plusieurs véhicules / bases comprenant de l'eau | de 5 % à 60 % m/m |
et dans laquelle ledit amplificateur percutané est choisi dans le groupe constitué d'acide vaccénique, cis-vaccénique, linoléique, linolénique, élaïdique, oléique, pétrosélinique, érurique ou nervonique et/ou de n'importe lequel de leurs alcools correspondants, ou de dodécylazacyclo - 1 heptane-2-one ou de sulphoxydes tels que le diméthylsulfoxyde, le n-décylméthylsulphoxyde ou de diméthylacétamide, de diméthylformamide, de N,N-diméthyl-m-toluamide, ou de pyrrolidones telles que la 2-pyrrolidone et la N-méthyl-2-pyrrolidone.

3. Composition pharmaceutique selon la revendication 1 ou la revendication 2 qui comprend en outre un agent neutralisant / un agent ajustant le pH compris dans la plage allant de 0,0 % m/m à 2,0 % m/m.

4. Composition pharmaceutique selon l'une quelconque des revendications 1 à 3 dans laquelle l'amplificateur percutané est du diméthylacétamide.

5. Composition pharmaceutique selon l'une quelconque des revendications 1 à 4 dans laquelle ledit tensio-actif est choisi dans le groupe constitué
de tout tensio-actif hydrophile ou lipophile acceptable d'un point de vue pharmaceutique ou un mélange de ceux-ci, de produits de réaction d'huiles végétales naturelles ou hydrogénées et d'éthylène glycol tels que des huiles végétales naturelles ou hydrogénées glycolées de polyoxyéthylène telles que des huiles de ricin naturelles ou hydrogénées glycolées de polyoxyéthylène ; d'esters d'acides gras de polyoxyéthylène sorbitanne, par exemple des esters mono et trilaurylique, pamitylique, stéarylique et oléylique, de copolymères en bloc de polyoxyéthylène-polyoxypropylène, d'esters d'acides gras de polyoxyéthylène, de mono- et di-esters d'acides gras de propylène glycol tels que le dicaprylate de propylène glycol, le dilaurate de propylène glycol, l'hydroxystéarate de propylène glycol, l'isostéarate de propylène glycol, le laurate de propylène glycol, le ricinoléate de propylène glycol, le stéarate de propylène glycol ; de tensio-actifs lipophiles tels que les produits de transestérification de triglycérides d'huiles végétales naturelles et de polyalkylène-polyols, d'esters d'acides gras de sorbitanne, de monoglycérides tels que le monooléate de glycérol, le monopalmitate de glycérol et le monostéarate de glycéryl.

6. Composition pharmaceutique selon l'une quelconque des revendications 1 à 5 dans laquelle ledit gélifiant / agent de solidification est choisi dans le groupe constitué :
de matériels polymères synthétiques ou semi-synthétiques, de polyacrylate et de résines co-polymères de polyacrylate telles que les résines d'acide polyacrylique et d'acide polyacrylique / d'acide méthacrylique, de cellulose et des dérivés de la cellulose comprenant les alkyl-celluloses telles que les méthyl-, éthyl- et propyl-celluloses ; les hydroxyalkyl-celluloses telles que l'hydroxypropyl-cellulose, l'hydroxypropyl-alkyl-cellulose telle que l'hydroxypropyl-méthyl-cellulose, les celluloses acylées telles que les acétates de cellulose, les acétates-phthalates de cellulose et leurs sels tels que la carboxyméthylcellulose de sodium ; de résines polyvinyliques comprenant les acétates de polyvinyle et les alcools aussi bien que d'autres matériels polymères comprenant des alginates tels que l'acide alginique et ses sels tels que l'alginate de sodium et l'alginate de propylène glycol.

7. Composition pharmaceutique selon la revendication 2 dans laquelle l'eau est présente dans la plage allant de 1 % à 15 % m/m.

8. Composition pharmaceutique selon l'une quelconque des revendications 1 à 7 dans laquelle l'eau est présente dans la plage allant de 9 % à 11 %.

9. Composition pharmaceutique selon la revendication 8 dans laquelle l'eau est présente dans la plage allant de 9,5 % à 10,5 % m/m.

10. Composition pharmaceutique selon l'une quelconque des revendications 3 à 9 dans laquelle ledit agent neutralisant est choisi dans le groupe constitué :
de bicarbonate de sodium, d'hydroxyde de sodium, d'hydroxyde de potassium, de borax, de phosphate disodique et de phosphate monosodique, d'amines organiques polaires telles que la diéthylamine, la diisopropanolamine, la triéthylamine et la triéthanolamine.

11. Composition pharmaceutique selon l'une quelconque des revendications 1 à 10 dans laquelle ledit véhicule / ladite base est choisi dans le groupe constitué :
d'alcanols inférieurs (de C₁₋₅) acceptables d'un point de vue pharmaceutique, particulièrement l'éthanol ; de macrogols solubles dans l'eau comme le glycol de polyéthylène ayant un poids moléculaire moyen compris entre 200 et 600 ; de carbonate de 1,2-propylène, de propane-1,2-diol et de glycol de 1,2-propylène ; de triacétate de glycérol ou de (1,2,3)-triacétine ; de cétones inférieures, particulièrement l'acétone et le 1,2,3-propanetriol, d'alkyl C₁₋₅ ou de tétrahydrofurfuryl acceptables d'un point de vue pharmaceutique ; d'éther di- ou partiel d'un mono- ou polyoxy-alkanediol de faible poids moléculaire ; de triglycérides d'acides gras, de préférence des triglycérides d'acides gras à chaîne moyenne ; d'huiles végétales telles que les huiles de noix de coco, l'huile d'olive, l'huile de ricin et autres dérivés ; d'oléate d'éthyle, de graisses, d'huiles et de cires d'origine animale comme la cire d'abeilles, de spermaceti, de graisse de laine, de cires d'origine végétale ou d'origine minérale comme la paraffine dure, molle et liquide.

12. Composition pharmaceutique selon l'une quelconque des revendications 1 à 11 qui est sous la forme d'un gel aqueux topique.

13. Composition pharmaceutique selon l'une quelconque des revendications 1 à 11 qui est sous la forme d'une émulsion d'huile dans l'eau ou d'une émulsion d'eau dans l'huile ou de micro-émulsion ou de crème.

14. Composition pharmaceutique selon l'une quelconque des revendications 1 à 11 qui est sous la forme d'une solution pour une application topique.

15. Composition pharmaceutique selon l'une quelconque des revendications 1 à 11 qui est sous la forme d'un onguent.

16. Composition pharmaceutique selon l'une quelconque des revendications 1 à 11 qui est sous la forme d'une formule aérosol.

17. Un procédé pour la production d'une composition pharmaceutique thérapeutique anti-inflammatoire et analgésique pour une utilisation topique qui comprend les étapes suivantes consistant à :
a) mélanger un amplificateur percutané de 0,5 m/m à 30 % avec 2,5% à 30% m/m d'un ou plusieurs véhicules ou bases ;
dans lequel ledit amplificateur percutané est choisi dans le groupe constitué d'acide vaccénique, cis-vaccénique, linoléique, linolénique, élaïdique, oléique, pétrosélinique, érurique ou nervonique et/ou de n'importe lequel de leurs alcools correspondants, ou de dodécylazacyclo - 1 heptane-2-one ou de sulphoxydes tels que le diméthylsulfoxyde, le n-décylméthylsulphoxyde ou de diméthylacétamide, de diméthylformamide, de N,N-diméthyl-m-toluamide, ou de pyrrolidones telles que la 2-pyrrolidone et la N-méthyl-2-pyrrolidone.
b) ajouter au mélange de l'étape a), de 0,1 % m/m à 10 % m/m de nimésulide puis mélanger le mélange jusqu'à ce qu'il soit complètement dissout ;
c) mélanger séparément de 0,5 % m/m à 12 % m/m d'un surfactant, de 0,2 % m/m à 50 % m/m d'un gélifiant/agent épaississant et de 2,5 % m/m à 30 % m/m d'un ou plusieurs véhicules ou bases et à mélanger le mélange entier dans un homogénéisateur pour obtenir un mélange homogène ;
d) ajouter le mélange obtenu dans l'étape b) au mélange homogénéisé obtenu dans l'étape c) en mélangeant pour obtenir la composition souhaitée pour l'utilisation analgésique.

18. Un procédé selon la revendication 17 dans lequel un agent neutralisant ou un agent ajustant le pH est ajouté à la composition dans l'étape d) pour neutraliser ou ajuster le pH du mélange.

19. Un procédé selon la revendication 18 dans lequel ledit agent neutralisant /agent ajustant le pH est ajouté dans un intervalle allant de 0,0 % m/m à 2,0 % m/m.
